Europäisches Patentamt

European Patent Office

Office européen des brevets

(11) Publication number: **0 058 993**
**B1**

(12) **EUROPEAN PATENT SPECIFICATION**

(45) Date of publication of patent specification: **09.05.90**

(21) Application number: **82101407.3**

(22) Date of filing: **24.02.82**

(51) Int. Cl.⁵: **A 61 K 37/04** // A61K35/16

(54) **Process for heat treatment of aqueous solution containing cold insoluble globulin.**

(30) Priority: **25.02.81 JP 27448/81**

(43) Date of publication of application:
**01.09.82 Bulletin 82/35**

(45) Publication of the grant of the patent:
**09.05.90 Bulletin 90/19**

(84) Designated Contracting States:
**AT BE CH DE FR GB LI LU NL SE**

(56) References cited:
**EP-A-0 018 561**
**GB-A-1 230 127**
**JP-A-5 359 018**
**US-A-4 305 871**
**BIOLOGICAL ABSTRACTS, vol. 74, no. 2, 1982,**
**no. 7751; D.G. WALLACE et al.: "Biological**
**activity and conformational stability of the**
**domains of plasma fibronectin" & ARCH**
**BIOCHEM BIOPHYS 212(2): 515-524. 1981**
**CHEMICAL ABSTRACTS, vol. 97, no. 4, July 26,**
**1982,no. 28581s, Columbus, Ohio, US; & JP - A -**
**82 35 520 (GREEN CROSS CORP.) 26-02-1982**

(73) Proprietor: **GREEN CROSS CORPORATION**
**15-1, Imabashi-1-chome Higashi-ku Osaka-shi**
**Osaka 541 (JP)**

(72) Inventor: **Ohmura, Takao**
**15-21, Aobadai-2-chome**
**Takarazuka-shi (JP)**
Inventor: **Hirao, Yutaka**
**14-1-606, Terauchi-2-chome**
**Toyonaka-shi Osaka-fu (JP)**
Inventor: **Funakoshi, Satoshi**
**16-5, Aoyama-1-chome**
**Katano-shi (JP)**

(74) Representative: **Vossius & Partner**
**Siebertstrasse 4 P.O. Box 86 07 67**
**D-8000 München 86 (DE)**

Courier Press, Leamington Spa, England.

## Description

This invention relates to a process for heat treatment of an aqueous solution containing cold insoluble globulin.

Cold insoluble globulin (CIG) has hitherto been known, inter alia, as "large external trypsin sensitive protein (LETS)", "cell surface protein (CSP)", "cell adhesion factor (CAF)" or "opsonic $\alpha_2$ surface binding glycoprotein (O-$\alpha_2$ SBG)". Recently it has generally come to be known as CIG or fibronectin. It is a glycoprotein having a molecular weight of 440,000 which occurs in, besides plasma, mesenchymal cells such as fibroblasts or basilar membranes such as the epidermis. With regard to the other known physicochemical properties of CIG, it may be mentioned that its mobility is that of $\alpha_2$ globulin, its isoelectric point is 5.0, its molecular extinction coefficient A $_{1cm}^{1\%}$ 280 nm is 12.9—13.0, $S_{20'w}$ is 11—14S and its carbohydrate content is 5%.

In the coagulation of blood, bonding between δ-chains of fibrin is accelerated by the transglutaminase action of blood coagulation factor XIII, thereby producing crosslinked fibrin. In this instance, CIG produces the crosslinking between α-chains of fibrin by catalytic action of the same factor XIII, thereby also making the blood coagulation more complete; CIG also has the function of effecting adhesion or bonding between cells and cell sustentacular tissue, and hence has the pharmacological efficacy to promote wound healing. The pharmacological advantages so far reported include treatment for septic shock, and treatment of infective diseases on the basis of its enhancing the opsonic action of phagocytes. Moreover, it is known that CIG has anticancer and antileukemia activity due to its action of enhancing the intercellular adhesive properties and causing necrosis of cancer cells. Accordingly great hopes are being placed in the chemical effects of CIG as a medicine.

CIG is obtained, inter alia, by separation from fibroblast culture fluids or fractions of plasma protein, and when thus separated there is a danger of contamination by viruses or other organisms. More particularly, when CIG is obtained by fractionation of plasma protein, there is a risk of hepatitis virus contamination. This is a serious problem. With regard to hepatitis B in particular, although much progress has been made towards the prevention of hepatitis B infections caused by plasma fraction preparations by subjecting to fractionation only the plasma that has proved to be negative to the virus (hepatitis B virus: HBV) after examination of the plasma material by a highly-sensitive detection method such as radioimmunoassay or the reversed passive hemagglutination test, total prevention is virtually impossible. In other words, even when the test for hepatitis B surface antigen (HBsAg) is negative, up to $10^8$ HBsAg particles may still be present in 1 ml plasma. This corresponds to $10^5$ HBV particles pro ml plasma.

In the plasma fractionation preparations, which entailed a definite risk of an outbreak of hepatitis, the first success was achieved in inactivating the hepatitis virus in albumin preparations by heat treatment at 60°C for 10 hours. It has been verified recently that the infectivity of the hepatitis virus can be reduced by a factor of 10,000 by this treatment.

In the case of CIG preparations for clinical applications, likewise, it is important that said preparations have been subjected to heat treatment at 60°C for 10 hours in order to prevent a hepatitis infection. But when the heat treatment is carried out in conventional aqueous solutions such as a physiological saline solution, the greater part of the CIG loses its activity or its protein molecules undergo denaturation.

JP—A—5 3—59 018 describes a process for preparing factor XIII concentrate. To inactivate any contaminating hepatitis viruses, this protein preparation is heated to about 55°C to 65°C for 9 to 11 hours in the presence of 10 to 20% w/v of a neutral amino acid, monosaccharide or sugar alcohol.

EP—A—0018 561 discloses a process for producing a hepatitis-free and essentially fibrinogen-free coagulation or fibrinolysis factor of the group consisting of factors II, VIII, XIII, antithrombin III and plasminogen by heating the protein preparations to about 60°C to 80°C in the presence of 1.0 to 2.5 mole/l of glycine and 40 to 60% w/v of sucrose at a pH of between 6.5 and 8.

The problem underlying the present invention is to provide a process for inactivating hepatitis virus in CIG by heat treatment without causing significant denaturation. The solution of this problem is based on the finding that the heat stability of CIG is markedly increased by adding a neutral amino acid, a monosaccharide, a disaccharide and/or a sugar alcohol (these are hereinafter referred to generically as principal stabilizers), and even further increased by the additional presence of a specified salt of a carboxylic acid (hereinafter simply referred to as auxiliary stabilizer).

Thus, the present invention relates to a process for the inactivation of hepatitis virus in cold insoluble globulin by heat treatment, which comprises heating an aqueous solution containing the cold insoluble globulin at 50 to 80°C for 5 to 20 hours in the presence of 10 to 62% W/V of at least one neutral amino acid, monosaccharide, disaccharide and/or sugar alcohol, and 10 to 30% W/V of at least one physiologically acceptable salt of a hydrocarbon carboxylic acid or hydroxyhydrocarbon carboxylic acid having 3—10 carbon atoms.

As the aqueous solutions containing CIG of the present invention, there may in general be used an aqueous solution containing CIG originating from a plasma protein fraction, fibroblasts or culture liquids thereof. The aqueous solution containing CIG to be used may be at any stage of purification, ranging from unpurified to purified aqueous solutions. Advantageously, however, an aqueous solution at a partly purified or purified stage is subjected to the heat treatment, and the CIG concentration is preferably 0.1—10% W/V.

# EP 0 058 993 B1

In the present context, the expression "% W/V" means percentage of solute by weight per solution by volume.

The pH of said aqueous solution is generally 5—10, and is advantageously adjusted to 6.5—8.5 with a buffer solution of a low salt concentration.

Examples of the principal stabilizers to be used according to the present invention include glycine, alanine, valine, leucine and isoleucine as neutral amino acids (i.e. monoamino monocarboxylic acids); glucose, mannose, galactose and fructose as monosaccharides; sucrose, maltose and lactose as disaccharides; and mannitol, sorbitol and xylitol as sugar alcohols; but they are not limited to the above examples. The amount of said principal stabilizer to be added is 10—50% W/V, in practice preferably 10—30% W/V.

The carboxylic acid having 3—10 carbon atoms from which the salts used as auxiliary stabilizer according to the present invention are hydrocarbon radicals substituted with carboxyl groups; the hydrocarbon group being either saturated or unsaturated. Examples of such hydrocarbon groups include alkyl, aryl (e.g. phenyl) and aralkyl groups, which may have a hydroxyl group. The number of carboxyl groups in said carboxylic acid may be one or several, preferably one or two. Though the salt of said organic carboxylic acid is not specifically restricted as long as it is physiologically acceptable, alkali metal salts (e.g. the sodium salt and potassium salt) and alkaline earth metal salts (e.g. the calcium salt) are preferred. The sodium salt and potassium salts are especially preferable.

Preferred examples of said carboxylic acid salts are physiologically acceptable salts, in particular alkali metal salts (sodium salts and potassium salts), of propanoic acid, butanoic acid, pentanoic acid, caprylic acid, caproic acid, malonic acid, succinic acid, glutaric acid, adipic acid, citric acid and mandelic acid.

The amount of carboxylic acid salt to be added is 10—30% W/V, in practice preferably 10—20% W/V.

The heat treatment is carried out at a temperature and for a period of time sufficient to inactivate the hepatitis virus but retain the activity of CIG, in general for 5—20 hours, preferably 10 hours at 50°—80°C, preferably at 60°C.

The stabilizer remaining in the aqueous solution after the heat treatment to inactivate the hepatitis virus can be removed by dialysis, especially dialysis under reduced pressure accompanied by concentration, or as the supernatant from the precipitation treatment with ammonium sulfate.

This invention will be illustrated in more detail with reference to the following examples.

## Reference Example

Pooled human blood plasma from adults is fractionated with ethanol, and CIG is purified by a suitable method from fraction I thus obtained. For example, according to the method of Matsuda et al., Ann. N.Y. Acad. Sci., *312*, 74, 1978, fraction I is dissolved in a 0.055M sodium citrate buffer solution, pH 6.0, and 0.01M of EACA (ε-aminocaproic acid) and 10 units/ml of Aprotinin (trypsin inhibitor) are added to the solution to prevent degradation of the CIG by plasmin. After plasmin and plasminogen have been removed using lysine-Sepharose® prepared by coupling lysine to Sepharose® (Cross-linked, agarose, sold by Pharmacia, Sweden) activated with cryanogen bromide, 10 units/ml of heparin are added, the solution is kept at 0°—2°C for 48 hours, and the precipitate is collected.

The precipitate is washed in turn with a 0.05M phosphate buffer solution, pH 6.0, and 0.05M citrate buffer solution containing 1M glycine and a 6.5% ethanol to remove soluble protein. Thereafter a 0.055M citrate buffer solution pH 6.35, is added and the mixture is brought to room temperature to dissolve the precipitate. An analogous procedure of keeping at 0—2°C and separating and washing the precipitate is repeated three times, and a cryofibrinogen fraction containing CIG is obtained as the last precipitate.

The cryofibrinogen (precipitate) is dissolved in twice as much 0.05M tris-phosphate buffer solution, pH 7.0, by weight as the raw material fraction I which was used to collect the cryofibrinogen, and is adsorbed on DEAE-Sephadex® (cross-linked dextran with diethylaminoethyl groups, sold by Pharmacia, Sweden) equilibrated with a 0.05M Tris-phosphate buffer solution, pH 7.0, and, after the contaminating protein has been removed by washing with the same buffer solution and with a 0.09M Tris-phosphate buffer solution, pH 7.0, the CIG is eluted with a 0.2M Tris-phosphate buffer solution, pH 7.0.

## Example 1

To 1 l of an aqueous solution containing 30 mg/ml CIG dissolved in a 0.05 M Tri-phosphate buffer solution (pH 8.0) were added 1 kg of sucrose and 190 g of sodium citrate. After being stirred thoroughly the mixture was heated at 60°C for 10 hours. After cooling, the mixture was dialyzed against a 0.9% sodium chloride solution and then centrifuged. A clear solution was obtained.

Quantitative determination of CIG was made on the CIG thus obtained by single radial immunodiffusion. The recovery of CIG was 100%.

## Experimental Example 1

The heat-treated CIG obtained in Example 1 and 2 was subjected to gel filtration using Sephadex® G-200 with a 0.05M Tris-phosphate buffer solution, pH 7.0. The fractions containing CIG were collected and saturated to 40% with ammonium sulfate. The precipitates formed were collected and dialyzed against a 0.05M Tris-phosphate buffer solution (pH 7.0). A purified CIG solution of 1% protein concentration was obtained.

Immuno-electrophoresis was carried out on this solution using antihuman whole serum (rabbit). No precipitin line other than that of CIG was observed.

Experimental Example 2

The purified CIG obtained in Experimental Example 1 was employed to immunize a rabbit. Antihuman CIG (heat-treated) antiserum (anti-CIG (h)) was thus obtained. Separately, CIG which had been purified according to a conventional method without heat treatment was employed to immunize a rabbit, and antiserum (anti-CIG (n)) was obtained. The micro-Ouchterlony technique was carried out using both of the above antiseras. The result revealed that the two kinds of purified CIG antigen, with and without heat treatment, when tested against anti-CIG (h) as well as anti CIG (n) gave precipitin lines which fused into one another completely and did not show any spur or partial spur.

This showed that no denaturation was observed such as would cause excess or deficiency of antigenicity of heat-treated CIG.

Experimental Example 3

An experiment was performed for the purpose of ascertaining the stabilizing effect according to the present invention. In this experiment various kinds of stabilizers indicated in Tables 1 and 2 were added in various amounts to 1 l of the same aqueous solution as employed in Example 1 containing 30 mg/ml of CIG. The solution was heated at 60°C for 10 hours. The remainder of the CIG value in percent is shown in Table 1 and Table 2.

The results reveal that the heat stability of CIG is increased by the addition of the principal stabilizer (Table 1). The heat stability of CIG is further increased by the addition of the auxiliary stabilizer in addition to the principal stabilizer (Table 2).

TABLE 1

| Principal Stabilizer | Concentration (W/V %) | CIG remaining value (%) |
|---|---|---|
| Glycine | 10 | 35 |
| | 15 | 62 |
| | 20 | 70 |
| Mannitol | | 28 |
| | 15 | 46 |
| | 20 | 60 |
| Glucose | 10 | 16 |
| | 30 | 38 |
| | 50 | 51 |
| Sucrose | 10 | 43 |
| | 30 | 75 |
| | 50 | 83 |
| None | | 0 |

# EP 0 058 993 B1

TABLE 2

| Principal stabilizer and concentration (W/V%) | Auxiliary stabilizer | Concentration (W/V %) | CIG remaining value (%) |
|---|---|---|---|
| Sucrose 50 | Sodium citrate | 0 | 83 |
| | | 10 | 88 |
| | | 15 | 92 |
| | | 20 | 100 |
| | Sodium caprylate | 0 | 83 |
| | | 10 | 90 |
| | | 15 | 93 |
| | | 20 | 98 |
| Glycine 20 | Sodium citrate | 0 | 70 |
| | | 10 | 75 |
| | | 15 | 77 |
| | | 20 | 88 |
| | Sodium mandelate | 0 | 70 |
| | | 10 | 78 |
| | | 15 | 74 |
| | | 0 | 83 |
| None | None | | 0 |

## Claims

1. A process for inactivation of hepatitis virus in cold insoluble globulin by heat treatment, characterized in that an aqueous solution containing the cold insoluble globulin is heated to 50 to 80°C for 5 to 20 hours in the presence of 10 to 62% W/V of at least one neutral amino acid, monosaccharide, disaccharide and/or sugar alcohol, and 10 to 30% W/V of at least one physiologically acceptable salt of a hydrocarbon carboxylic acid or hydroxyhydrocarbon carboxylic acid having 3 to 10 carbon atoms.

2. The process according to Claim 1, wherein the neutral amino acid is glycine, alanine, valine, leucine or isoleucine.

3. The process according to Claim 1, wherein the monosaccharide is glucose, mannose, galactose or fructose.

4. The process according to Claim 1, wherein the disaccharide is sucrose, maltose or lactose.

5. The process according to Claim 1, wherein the sugar alcohol is mannitol, sorbitol or xylitol.

6. The process according to Claim 1, wherein the amount of neutral amino acid, monosaccharide, disaccharide or sugar alcohol is 10—30% W/V.

7. The process according to Claim 1, wherein the aqueous solution contains the cold insoluble globulin in a concentration of 0.1 to 10% W/V.

8. The process according to Claim 1, wherein the heat treatment is carried out in a buffered aqueous solution at a pH of 5—10.

9. The process according to Claim 1, wherein the physiologically acceptable salt is a salt of propanoic acid, butanoic acid, pentanoic acid, caprylic acid, caproic acid, malonic acid, succinic acid, glutaric acid or adipic acid.

5

10. The process according to Claim 1, wherein the physiologically acceptable salt is a salt of citric acid or mandelic acid.

11. The process according to Claim 1, wherein the salt is a sodium or potassium salt.

**Patentansprüche**

1. Verfahren zur Inaktivierung von Hepatitis-Virus in kaltunlöslichem Globulin durch Hitzebehandlung, dadurch gekennzeichnet, daß eine wäßrige Lösung, die das kalt-unlösliche Globulin enthält, in Gegenwart von 10 bis 62% Gew./Vol. mindestens einer neutralen Aminosäure, eines Monosaccharids, Disaccharids und/oder Zuckeralkohols und von 10 bis 30% Gew./Vol. mindestens eines physiologisch verträglichen Salzes einer Kohlenwasserstoff-Carbonsäure oder einer Hydroxykohlenwasserstoff-Carbonsäure mit 3 bis 10 Kohlenstoffatomen 5 bis 20 Stunden auf 50 bis 80°C erhitzt wird.

2. Verfahren nach Anspruch 1, wobei die neutrale Aminosäure Glycin, Alanin, Valin, Leucin oder Isoleucin ist.

3. Verfahren nach Anspruch 1, wobei das Monosaccharid, Glucose, Mannose, Galactose oder Fructose ist.

4. Verfahren nach Anspruch 1, wobei das Disaccharid, Saccharose, Maltose oder Lactose ist.

5. Verfahren nach Anspruch 1, wobei der Zuckeralkohol Mannit, Sorbit oder Xylit ist.

6. Verfahren nach Anspruch 1, wobei die Menge der neutralen Aminosäure, des Monosaccharids, Disaccharids oder Zuckeralkohols 10 bis 30% Gew./Vol. beträgt.

7. Verfahren nach Anspruch 1, wobei die wäßrige Lösung das kalt-unlösliche Globulin in einer Konzentration von 0,1 bis 10% Gew./Vol. enthält.

8. Verfahren nach Anspruch 1, wobei die Hitzebehandlung in einer gepufferten wäßrigen Lösung bei einem pH-Wert von 5 bis 10 durchgeführt wird.

9. Verfahren nach Anspruch 1, wobei das physiologisch verträgliche Salz ein Salz von Propionsäure, Buttersäure, Pentansäure, Caprylsäure, Capronsäure, Malonsäure, Bernsteinsäure, Glutarsäure oder Adipinsäure ist.

10. Verfahren nach Anspruch 1, wobei das physioligisch verträglich Salz ein Salz von Citronensäure oder Mandelsäure ist.

11. Verfahren nach Anspruch 1, wobei das Salz ein Natrium- oder Kaliumsalz ist.

**Revendications**

1. Procédé d'inactivation du virus de l'hépatite dans une globuline insoluble à froid par traitement thermique, caractérisé en ce qu'on chauffe une solution aqueuse contenant la globuline insoluble à froid à 50 à 80°C pendant 5 à 20 heures en présence de 10 à 62% P/V d'au moins un aminoacide neutre, un monosaccharide, un disaccharide et/ou un alcool de sucre, et 10 à 30% P/V d'au moins un sel physiologiquement acceptable d'un acide hydrocarbure carboxylique ou d'un acide hydroxyhydrocarbure carboxylique en $C_3$ à $C_{10}$.

2. Procédé suivant la revendication 1, dans lequel l'aminoacide neutre est la glycine, l'alanine, la valine, la leucine ou l'isoleucine.

3. Procédé suivant la revendication 1, dans lequel le monosaccharide est le glucose, le mannose, le galactose ou le fructose.

4. Procédé suivant la revendication 1, dans lequel le disaccharide est le saccharose, le maltose ou le lactose.

5. Procédé suivant la revendication 1, dans lequel l'alcool de sucre est le mannitol, le sorbitol ou le xylitol.

6. Procédé suivant la revendication 1, dans lequel la quantité d'aminoacide neutre, de monosaccharide, de disaccharide ou d'alcool de sucre est de 10 a 30% P/V.

7. Procédé suivant la revendication 1, dans lequel la solution aqueuse contient la globuline insoluble froide à une concentration de 0,1 à 10% P/V.

8. Procédé suivant la revendication 1, dans lequel le traitement thermique est effectué dans une solution aqueuse tamponnée à un pH de 5 à 10.

9. Procédé suivant la revendication 1, dans lequel le sel physiologiquement acceptable est un sel de l'acide propanoïque, de l'acide butanoïque, de l'acide pentanoïque, de l'acide caprylique, de l'acide caproïque, de l'acide malonique, de l'acide succinique, de l'acide glutarique ou de l'acide adipique.

10. Procédé suivant la revendication 1, dans lequel le sel physiologiquement acceptable est un sel de l'acide citrique ou de l'acide mandélique.

11. Procédé suivant la revendication 1, dans lequel le sel est un sel de sodium ou de potassium.